# EUROPÄISCHE PATENTSCHRIFT

(11) **EP 1 125 554 B1**
(45) Veröffentlichungstag und Bekanntmachung des Hinweises auf die Patenterteilung: **03.06.2009**
(21) Anmeldenummer: 01103928.6
(22) Anmeldetag: 16.02.2001
(51) Int. Cl.: A61B 17/135

(54) **Venenstaugerät**
Vein occluding apparatus
Appareil d'occlusion veineuse

(30) Priorität: 17.02.2000 DE 10007231
(43) Veröffentlichungstag der Anmeldung: 22.08.2001
(73) Patentinhaber: Mach, Jakub, Dr., 93437 Furth im Wald (DE)
(72) Erfinder: Mach, Jakub, Dr., 93437 Furth im Wald (DE)
(74) Vertreter: TBK-Patent

(56) Entgegenhaltungen:
- US-A- 5 584 853
- US-A- 5 911 735

## Beschreibung

Die vorliegende Erfindung betrifft einen Venenstaugerät gemäß dem Oberbegriff des Patentanspruchs 1 und insbesondere ein Venenstaugerät für das Anlegen an den Oberarm eines Patienten bei Blutentnahmevorgängen.

Zum Alltag der Arbeit von Ärzten in Krankenhäusern, sowie in Arztpraxen zählt unter anderem die Blutentnahme. Um hierfür die Venen des Oberarms eines Patienten zu stauen, wird ein venöser Stauschlauch benutzt. Es handelt sich hierbei um ein flexibles Band mit einem kleinen Verschluss. Dieser bekannte Stauschlauch wird um den Oberarm eines Patienten geschlungen und zugezogen, wodurch die Venen abgedrückt werden und das darin befindliche Blut gestaut wird. Dies wiederum bewirkt ein Hervorquellen der Venen, wodurch der Arzt genauer eine Kanüle zur Blutentnahme einstechen und somit das Blut entnehmen kann.

Diese Methode hat jedoch den Nachteil, dass ein behandelnder Arzt keine Kontrolle darüber hat, mit welchem Druck der verwendete Stauschlauch auf den Oberarm in Abhängigkeit von der Zugkraft im Stauschlauch einwirkt. Dieser Nachteil wird noch dadurch verstärkt, dass es sich bei den herkömmlichen Stauschläuchen um flexibles Material handelt, welches sich bei entsprechenden Zugkräften dehnt und somit ein äußerst schwammiges Kraftgefühl für den Arzt vermittelt.

Aufgrund dieser Kontrollunsicherheiten geschieht es häufig, dass nicht nur die Venen sondern auch die tiefer liegenden Arterien des zu behandelnden Patienten abgedrückt werden, so dass in diesem Fall ein Effekt der Überfüllung der Venen und damit ein Sichtbarwerden der Venen nicht mehr möglich ist.

Angesichts dieses Stands der Technik besteht die Aufgabe der Erfindung darin, ein Venenstaugerät, wie es beispielsweise für die Blutentnahme Verwendung findet, zu schaffen, welches exakter betätigbar, ist, um den Effekt der Überfüllung der Venen zu garantieren.

Diese Aufgabe wird durch ein Venenstaugerät mit den Merkmalen des Patentanspruchs 1 gelöst. Die Erfindung besteht demzufolge darin, das Venenstaugerät als eine Druckschlauchmanschette mit einer daran angeschlossenen Druckquelle auszubilden und zusätzlich ein Druckeinstell-Hilfsmittel vorzusehen, mittels welchem der Manschetteninnendruck erfasst und/oder auf einen vorbestimmten oder vorbestimmbaren Wert eingestellt werden kann. Dieser Wert entspricht einem Druck, der ein Abdrücken einer Vene bewirkt, gleichzeitig jedoch den Strömungsquerschnitt einer Arterie zumindest teilweise erhält. Auf diese Weise lässt sich eine Blutströmung in der Arterie aufrecht erhalten, so dass die abgedrückte Vene sich allmählich durch das darin sich anstauende Blut aufbläht und somit für den Arzt besser sichtbar wird.

Für die Ausbildung des Druckeinstell-Hilfsmittels eignen sich unterschiedliche Einrichtungen. Das Druckeinstell-Hilfsmittel enthält eine Druckvorgabeeinhet und eine Regeleinheit. Gemäß Anspruch 2 ist die Regeleinhet ein manuell oder elektronisch einstellbares Überdruckventil, welches an der Druckschlauchmanschette oder der Druckquelle angeordnet ist. Diese Ausgestaltung hat den Vorteil, dass ohne größere technische Maßnahmen ein Überschreiten eines vorbestimmten oder vorbestimmbaren Werts ausgeschlossen ist.

Alternativ hierzu sieht der Patentanspruch 1 vor, das Druckeinstell-Hilfsmittel mit einem Drucksensor als Regeleinheit auszubilden, der an einem Mikrocomputer angeschlossen ist, an welchem der vorbestimmte oder vorbestimmbare Wert eingestellt wird. Diese Ausführungsform erleichtert die Bedienbarkeit des Geräts und gewährleistet eine exaktere Einstellung des Manschetteninnendrucks durch den Rückkopplungsregelvorgang zwischen dem Drucksensor, dem Mikrocomputer und der Druckquelle.

Eine Ausgestaltung des Druckeinstell-Hilfsmittels sieht gemäß dem Patentanspruch 6 die Anordnung einer einfachen Druckanzeige vor, die visuell oder akustisch das Erreichen des vorbestimmten oder vorbestimmbaren Werts signalisiert.

Weitere vorteilhafte Ausgestaltungen der Erfindung sind Gegenstand der übrigen Unteransprüche.

Die Erfindung wird nachstehend anhand eines bevorzugten Ausführungsbeispiels unter Bezugnahme auf die begleitenden Zeichnungen näher beschrieben.

Die Figur 1 zeigt die Prinzipskizze eines Venenstaugeräts gemäß einem bevorzugten Ausführungsbeispiel der Erfindung und

Fig. 2a, 2b zeigen die Konstruktion des Venenstaugeräts mit an der Druckmanschette zu einer Einheit integriertem Mikroprozessor und Druckquelle.

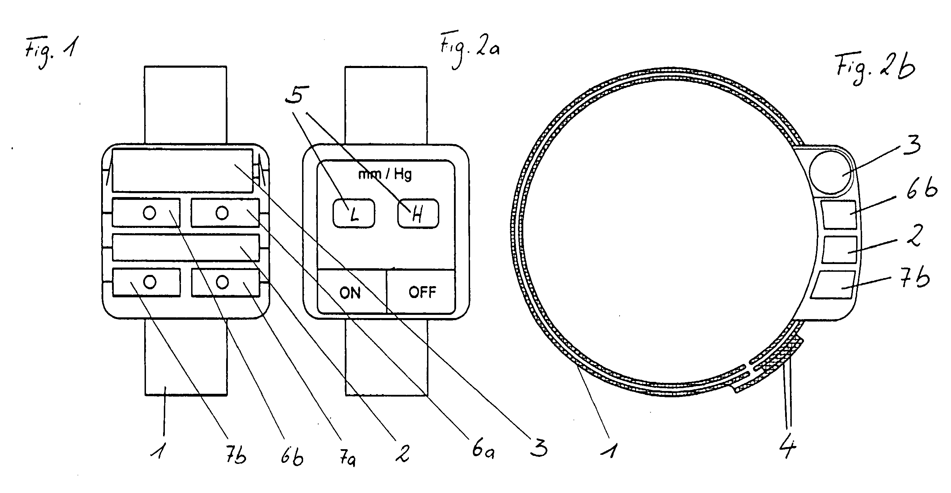

Gemäß der Figur 1 besteht das Venenstaugerät aus einer Druckschlauchmanschette 1 sowie einer Druckquelle 2, die über einen Verbindungskanal (nicht gezeigt) mit der Druckschlauchmanschette 1 fluidverbunden ist. Die Druckquelle 3 ist vorliegend als eine pneumatische Druckpumpe ausgebildet, welche durch einen vorzugsweise Batterie gespeisten Elektromotor 2 angetrieben wird. Alternativ kann natürlich auch eine manuell betätigte Druckpumpe beispielsweise ein Blasbalg vorgesehen sein.

Die Druckschlauchmanschette 1, besteht aus einem elastischen Schlauchmaterial, welches um den Oberarm eines Patienten geschlungen und zu einem geschlossenen Kreis fixiert werden kann. Hierfür hat die Druckschlauchmanschette 1 gemäß Fig. 2b an ihren sich gegenüberliegenden Enden Klettverschlüsse 4, die beim kreisförmigen Zusammenlegen der Manschette 1 zu einer festen Verbindung zusammenwirken.

Des weitern ist ein Druckeinstell-Hilfsmittel bestehend aus einer Druckvorgabeeinheit sowie einer Regeleinheit vorgesehen, die vorzugsweise mit der Druckquelle 3 zu einer Baueinheit integriert sind.

Die Druckvorgabeeinheit hat einen Mikrocomputer (nicht weiter gezeigt) sowie ein Eingabebauteil 5 beispielsweise eine Anzahl von Druckknöpfen, eine Wählscheibe oder einen Drehknopf, welches mit dem Mikrocomputer verbunden ist. So ist das Druckeinstell-Hilfsmittel kontinuierlich oder gerastet auf den vorbestimmten Druckwert verstellbar. Der Mikrocomputer ist ferner an die Regeleinheit in Form mindestens eines vorliegend zweier elektromagnetischer Uberdruckventile 6a, 6b sowie an die Druckquelle 3 angeschlossen. Optional kann eine Druckanzeige (nicht gezeigt) vorgesehen sein, die eine visuelle Überwachung des Druckaufbaus oder -abbaus innerhalb der Manschette 1 zulässt. Alternativ zu den vorstehend genannten elektromagnetischen Uberdruckventilen 6a, 6b ist die Anordnung eines Drucksensors (nicht dargestellt) innerhalb der Druckquelle 3 oder der Manschette 1 möglich, welcher mit dem Mikrocomputer elektrisch verbunden ist. Über diesen Drucksensor erhält der Mikrocomputer Informationen über den gegenwärtig vorherrschenden Druck, so dass ein Ist-Soll-Druckvergleich ausführbar ist.

Je nach Ausführungsform ist in dem Mikrocomputer eine Wertetabelle abgespeichert, in welcher zumindest zwei Druckwerte unterschiedlicher Höhe gelistet sind.

Es hat sich gezeigt, dass bei jüngeren Menschen der systolische Druck, d.h. jener Blutdruck innerhalb der Arterie bei einem Wert von ca. 100 bis 120 mm HG und der diastolische Druck, d.h. jener Blutdruck innerhalb der Vene bei einem Wert von ca. 60 bis 70 mm HG liegt. Bei älteren Menschen steigt der systolische Druck auf über 120 mm HG, während sich der diastolische Druck um ca. 90 mm HG bewegt. Es ergibt sich also jeweils ein Druckfenster von ca. 30 bis 40 mm HG, jedoch auf unterschiedlichem Niveau.

Bei einem herkömmlichen Blutdruckmessverfahren wird im allgemeinen ebenfalls um den Oberarm eines Patienten eine Druckmanschette gelegt und diese solange mit Druck beaufschlagt, bis die Strömung sowohl in der Vene als auch in der Arterie gesperrt wird. Anschließend wird der Druck innerhalb der Manschette langsam entspannt, solange, bis in der Arterie erneut eine Blutströmung entsteht. Der hierdurch gemessene Druck entspricht dem systolischen Druck. Bei weiterem Druckentspannen der Manschette beginnt auch in der Vene zu einem bestimmten Zeitpunkt erneut die Blutströmung, wobei der nunmehr vorherrschende Druck der diastolische Druck darstellt. Auf diese Weise ist für jeden Patienten individuell das vorstehend genannte Druckfenster bestimmbar. Dabei hat sich gezeigt, dass sich dieses Druckfenster bei jungen und alten Menschen, d.h. altersbedingt im wesentlichen gleichmäßig unterscheidet, so dass es ausreicht, zumindest zwei Druckwerte bezogen auf die verwendete Druckschlauchmanschette für alte und junge Patienten im Mikrocomputer abzuspeichern. Dabei ist das genannte Druckfenster groß genug, um individuelle Abweichungen beispielsweise im Falle von vorliegenden Krankheiten oder unterschiedlichen körperlichen Konstitutionen auszugleichen. In anderen Worten ausgedrückt werden zumindest zwei Werte in Abhängigkeit der Ausgestaltung der Druckschlauchmanschette abgespeichert, die zwei Drücken entsprechen, welche auf die Vene und die Arterie jeweils eine Druckkraft zwischen 60 und 120 vorzugsweise 80 mm HG und 100 mm Hg ausüben.

Selbstverständlich ist es auch möglich, den Mikrocomputer mit einer Blutdruckmessfunktion auszustatten, wodurch der systolische und der diastolische Druck für jeden zu behandelnden Patienten individuell gemessen werden kann und gleichzeitig jene zwei Drücke abgegriffen werden, die zu den entsprechenden Zeitpunkten innerhalb der Druckschlauchmanschette 1 vorherrschen. Anschließend wird ein Mittelwert zwischen den beiden Manschetteninnedrücken berechnet, der nunmehr den vorbestimmten Wert darstellt. Diese Ausführung ist jedoch nicht von dem Schutzumfang erfasst.

Eine weitere Vereinfachung, auch nicht von dem Schutzumfang erfasst, kann vorsehen, auf den Mikrocomputer komplett zu verzichten und im Falle der Anordnung von zwei Überdruckventilen, diese auf die zwei unterschiedliche Druckwerte einzustellen. Das Eingabebauteil ist dann so zu gestalten, dass es unmittelbar auf die 2 Ventile einwirkt und diese wahlweise in den Druckkreis zwischenschaltet.

Zur Funktion des erfindungsgemäßen Venenstaugeräts lässt sich Folgendes anführen:
Mit der vorab eingespeicherten Wertetabelle wird die Druckschlauchmanschette 1 an den Oberarm eines Patienten angelegt und anschließend in Abhängigkeit des Alters sowie ggf. der körperlichen Konstitution des Patienten der entsprechende Druckwert über die Druckvorgabeeinheit ausgewählt. Anschließend wird die Druckpumpe 3 durch die Druckvorgabeeinheit bzw. einen externen Ein/Aus-Schalter 7a, 7b gestartet, worauf diese den Innendruck innerhalb der Druckschlauchmanschette 1 anhebt. Die Druckveränderung wird vom Drucksensor oder von dem einen oder den zwei Überdruckventilen 6a, 6b registriert. Bei Erreichen des eingestellten Druckwerts, der als Druckschwellwert dient, gibt der Drucksensor oder das nunmehr sich öffnende Überdruckventil 6a, 6b ein Signal an den Mikrocomputer ab, worauf dieser die Druckpumpe 3 stoppt. Im Falle der Verwendung des Überdruckventils verhindert dieses gleichzeitig in jedem Fall das Überschreiten des voreingestellten Druckwerts und gleicht somit Regelungenauigkeiten sowie Regelzeitverzögerungen, welche beim Abstellen der Pumpe 3 ggf. auftreten können, aus.

In jenem Fall, nicht von dem Schutzumfang erfasst, wonach der Mikrocomputer zusätzlich mit einer Blutdruckmessfunktion ausgestattet ist, wird, nachdem die Druckschlauchmanschette 1 an den Oberarm des Patienten angelegt ist, in einem ersten Blutdruckmessverfahren die Pumpe 3 solange angetrieben, bis sowohl die Vene als auch die Arterie abgedrückt sind. Anschließend wird der Blutdruck nach dem eingangs beschriebenen Verfahren für jeden Patienten individuell gemessen.

Nachdem die in der Druckschlauchmanschette 1 vorherrschenden Innendrücke zum Zeitpunkt des systolischen und des diastolischen Drucks vom Drucksensor erfasst worden sind, bestimmt der Mikrocomputer einen Druckmittelwert aus den erfassten Innendrücken und treibt die Druckpumpe 3 solange an, bis dieser Druckmittelwert vom Drucksensor erneut erfasst und ein entsprechendes Signal beim Mikrocomputer eingegangen ist.

In diesem Fall ist die auf den Oberarm des zu behandelnden Patienten aufgebrachte Druckkraft exakt so groß, dass die Vene vollständig abgedrückt wird, während der Strömungsquerschnitt der Arterie zumindest teilweise aufrecht erhalten bleibt. Das durch die Arterie strömende Blut wird folglich ausschließlich in der Vene gestaut und bewirkt ein Aufquellen der Vene, wodurch diese für den behandelnden Arzt sichtbar wird.

Abschließend sei darauf hingewiesen, dass das vorstehend beschrieben Venenstaugerät auch unterschiedliche Abwandlungen aufweisen kann.

Beispielsweise kann das Druckeinstell-Hilfsmittel anstelle des vorstehend beschriebenen Überdruckventils 6a, 6b oder des Drucksensors eine einfache Druckanzeige aufweisen, über die der behandelnde Arzt den zu verwendenden Manschetteninnendruck ablesen kann. Es ist auch möglich, das/die Überdruckventile 6a, 6b oder einen Drucksensor mit einer solchen Druckanzeige zu kombinieren. Diese Kombination bietet sich insbesondere dann an, wenn das Venenstaugerät gleichzeitig eine Blutdruckmessfunktion besitzt.

Die Erfindung betrifft ein Venenstaugerät mit einer Druckschlauchmanschette und einer Druckquelle sowie einem Druckeinstell-Hilfsmittel zur Einstellung des Manschetteninnendrucks auf einen vorbestimmten oder vorbestimmbaren Wert. Dieser Druckwert bewirkt eine Druckkraft über die Druckschlauchmanschette auf den Oberarm eines der zu behandelnden Patienten, welche so groß ist, dass ein Abdrücken einer Vene bewirkt wird, der Strömungsquerschnitt einer Arterie jedoch zumindest teilweise erhalten bleibt. Der Druckwert wurde vorab durch Versuche für unterschiedliche Patienten (Alter, körperliche Konstitution, unterschiedliche Muskulatur, u.s.w.) bestimmt und in einer Tabelle abgespeichert.

## Patentansprüche

1. Venenstaugerät mit
- einer Druckquelle (3),
- einer Druckschlauchmanschette (1), die an die Druckquelle (3) angeschlossen ist und
- einem Druckeinstell-Hilfsmittel enthaltend eine Druckvorgabeeinheit zum Festlegen eines Werts für einen Innendruck innerhalb der Druckschlauchmanschette (1) und eine Regeleinheit zum Einregeln des Innendrucks innerhalb der Druckmanschette (1) auf den festgelegten Wert, **dadurch gekennzeichnet dass**
die Druckvorgabeeinheit folgende Elemente hat:
- einen mit einem Speicher ausgerüsteten Mikrocomputer, in welchem alters- und/oder körperliche Konstitutionsspezifische Druckwerte für den Innendruck innerhalb der Druckmanschette (1) in Form einer Wertetabelle abgespeichert sind und
- ein Eingabebauteil (5) zum manuellen Eingeben des Alters und/oder der körperlichen Konstitution des jeweiligen Patienten, wobei
- der Mikrocomputer Mittel aufweist, um den Wert des Innendruchs aus seinem Speicher in Abhängigkeit des manuell eingegebenen Alters und/oder der körperlichen Konstitution des Patienten auszulesen, bei dem das Venenstaugerät eine entsprechende Vene abdrückt, den Strömungsquerschnitt einer Arterie jedoch zumindest teilweise offen hält und um die Regeleinheit so zu steuern, dass der Innendruck innerhalb der Druckmanschette (1) auf dem ausgelesenen Wert gehalten wird.

2. Venenstaugerät nach Anspruch 1,
**dadurch gekennzeichnet, dass**
die Regeleinheit eine manuell oder elektronisch einstellbare Überdruckventilanordnung (6) ist.

3. Venenstaugerät nach Anspruch 1,
**dadurch gekennzeichnet, dass**
die Regeleinheit ein Drucksensor ist, der an den Mikrocomputer angeschlossen ist, um entsprechend der Drucksensorsignale die Druckquelle (3) zu regeln.

4. Venenstaugerät nach einem der vorstehenden Ansprüche,
**dadurch gekennzeichnet, dass**
das Druckeinstell-Hilfsmittel auf zumindest zwei unterschiedliche vor bestimmte Werte einstellbar ist.

5. Venenstaugerät nach einem der Ansprüche 1 bis 3,
**dadurch gekennzeichnet, dass** das Druckeinstell-Hilfsmittel
kontinuierlich oder gerastet auf den vor bestimmten Druckwert verstellbar ist.

6. Venenstaugerät nach Anspruch 1,
**dadurch gekennzeichnet, dass**
die Regeleinheit eine Druckanzeige hat, die zumindest das Erreichen des ausgelesenen oder vor bestimmten Druckwerts signalisiert.

7. Venenstaugerät nach einem der vorstehenden Ansprüche,
**dadurch gekennzeichnet, dass**
die Druckquelle (3) eine Pneumatikpumpe ist.

## Claims

1. Vein constriction device having
- a pressure source (3),
- a pressure hose cuff (1) which is connected to the pressure source (3), and
- a pressure adjustment aid which contains a pressure-prescribing unit for establishing a value for an internal pressure within the pressure hose cuff (1) and a control unit for adjusting the internal pressure within the pressure cuff (1) to the established value, **characterised in that**
the pressure-prescribing unit has the following elements:
- a microcomputer which is equipped with a memory, in which microcomputer pressure values, which are specific to age and/or physical constitution, for the internal pressure within the pressure cuff (1) are stored in the form of a value table and
- an input component (5) for manual input of the age and/or of the physical constitution of the respective patient,
- the microcomputer having means for reading out the value of the internal pressure from the memory thereof as a function of the manually input age and/or of the physical constitution of the patient, in whom the vein constriction device compresses a corresponding vein but keeps open at least partially the flow cross-section of an artery, and for controlling the control unit such that the internal pressure within the pressure cuff (1) is maintained at the value which is read out.

2. Vein constriction device according to claim 1,
**characterised in that**
the control unit is a manually or electronically adjustable excess-pressure valve (6).

3. Vein constriction device according to claim 1,
**characterised in that**
the control unit is a pressure sensor which is connected to the microcomputer for controlling the pressure source (3) corresponding to the pressure sensor signals.

4. Vein constriction device according to one of the preceding claims,
**characterised in that**
the pressure adjustment aid can be adjusted to at least two different predetermined values.

5. Vein constriction device according to one of the claims 1 to 3,
**characterised in that**
the pressure adjustment aid can be adjusted continuously or set to the predetermined pressure value.

6. Vein constriction device according to claim 1,
**characterised in that**
the control unit has a pressure display which signals the achievement of the read-out or predetermined pressure value.

7. Vein constriction device according to one of the preceding claims,
**characterised in that**
the pressure source (3) is a pneumatic pump.

## Revendications

1. Appareil d'occlusion veineuse, comprenant :
- une source de pression (3),
- une manchette en tuyau souple à pression (1), raccordée à la source de pression (3) et
- des moyens auxiliaires de réglage de pression, contenant une unité d'affectation de pression, pour fixer une valeur concernant une pression intérieure à l'intérieur de la manchette en tuyau souple à pression (1), et une unité de réglage, pour régler à la valeur fixée la pression intérieure à l'intérieur de la manchette à pression (1), **caractérisé en ce que**
l'unité d'affectation de pression comprend les éléments suivants :
- un micro-ordinateur, équipé d'une mémoire, dans laquelle sont mémorisées, sous forme d'un tableau de valeurs, des valeurs de pression spécifiques à l'âge et/ou à la constitution corporelle, pour la pression intérieure à l'intérieur de la manchette à pression (1), et
- un composant d'introduction (5), pour introduire manuellement l'âge et/ou la constitution corporelle du patient spécifique, où
- le micro-ordinateur présente des moyens, pour lire la valeur de la pression intérieure à partir de sa mémoire, en fonction de l'âge et/ou de la constitution corporelle du patient, dans lequel l'appareil d'occlusion veineuse comprime une veine correspondante, maintient cependant au moins partiellement ouverte la section transversale, offerte à l'écoulement, d'une artère, d'une artère, et pour commander l'unité de réglage de telle manière que la pression intérieure à l'intérieur de la manchette à pression (1) soit maintenue à la valeur lue.

2. Appareil d'occlusion veineuse selon la revendication 1,
**caractérisé en ce que**
l'unité de réglage est un agencement à soupape de surpression (6), réglable manuellement ou électroniquement.

3. Appareil d'occlusion veineuse selon la revendication 1,
**caractérisé en ce que**
l'unité de réglage est un capteur de pression, raccordé au micro-ordinateur, pour régler la source de pression (3) de manière correspondante aux signaux de capteur de pression.

4. Appareil d'occlusion veineuse selon l'une des revendications précédentes,
**caractérisé en ce que**
les moyens auxiliaires de réglage de pression sont réglables à au moins deux valeurs prédéterminées, différentes.

5. Appareil d'occlusion veineuse selon l'une des revendications 1 à 3,
**caractérisé en ce que**
les moyens auxiliaires de réglage de pression sont réglables, de façon continue ou discrète, à la valeur de pression prédéterminée.

6. Appareil d'occlusion veineuse selon la revendication 1,
**caractérisé en ce que**
l'unité de réglage comprend un affichage de pression, signalant au moins l'atteinte de la valeur de pression lue ou prédéterminée.

7. Appareil d'occlusion veineuse selon l'une des revendications précédentes, **caractérisé en ce que**
la source de pression (3) est une pompe pneumatique.
